# EUROPEAN PATENT APPLICATION

(11) **EP 4 625 431 A1**
(43) Date of publication of application: **01.10.2025**
(21) Application number: 25156108.0
(22) Date of filing: 05.02.2025
(51) Int. Cl.: G16H 30/40, G16H 50/20, G16H 50/70

(54) **DIAGNOSIS ASSISTANCE APPARATUS, RECORDING MEDIUM, AND DIAGNOSIS ASSISTANCE METHOD**

(30) Priority: 27.03.2024 JP 2024051994
(71) Applicant: NEC Corporation, 108-8001 Tokyo (JP)
(72) Inventor: OMI, Yasuo, Tokyo, 108-8001 (JP); SANO, Maki, Tokyo, 108-8001 (JP)
(74) Representative: Betten & Resch

(57) **Abstract**

In order to attain an object to improve accuracy in automatic determination of a subtype carried out on the basis of a pathological image, at least one processor included in a diagnosis assistance apparatus carries out: a first acquisition process of acquiring a first caption from a first learned model, the first learned model being constructed by machine learning so as to generate, in a case where a pathological image is inputted, a caption describing content of the pathological image in a predetermined format, the first caption describing, in the predetermined format, content of a first pathological image to be subjected to diagnosis; a first evaluation process of evaluating a first similarity which is a similarity between (i) content of at least a part of a plurality of findings which are accumulated in a database and which represent, in writing in the predetermined format, a respective plurality of pathological subtypes and (ii) content of the first caption; and an output process of outputting information pertaining to a subtype whose first similarity is evaluated to be the highest among those of the plurality of pathological subtypes. The information outputted in the output process is used for decision making in diagnosis by a doctor.

## Description

### CROSS REFERENCE TO RELATED APPLICATIONS

This application is based upon and claims the benefit of priority from Japanese Patent Application No. 2024-051994 filed on March 27, 2024, the disclosure of which is incorporated herein in its entirety by reference.

### Technical Field

The present disclosure relates to a diagnosis assistance apparatus, a recording medium, and a diagnosis assistance method.

### Background Art

Various technologies for supporting diagnosis carried out by a doctor with use of a medical image have been proposed. For example, Patent Literature 1 discloses an image retrieval apparatus that operates as follows. The image retrieval apparatus: receives an input of finding information on a query base image to derive a query image to which a finding has been added; derives an added finding feature amount for the added finding; and derives a query normal feature amount indicating an image feature for a normal region in the query base image. Further, with reference to an image database in which a plurality of reference images, which are associated with a reference finding feature amount and a reference normal feature amount, have been registered, the image retrieval apparatus derives a similarity between the query image and each of the plurality of reference images. Further, the image retrieval apparatus extracts, from the image database and on the basis of the similarity, a reference image that is similar to the query image as a similar image.

### Citation List

### [Patent Literature]

[Patent Literature 1]
International Publication No. WO 2023/276432

### Summary of Invention

### Technical Problem

A lesion (e.g., dermatitis and cancer) has numerous subtypes, into which the legion is categorized according to the properties of cells. In diagnosis of such a legion by a doctor, the doctor determines a subtype to which the legion belongs. However, conventional technologies as disclosed in Patent Literature 1 do not offer sufficient accuracy in deriving a feature. As such, the doctor refers to an extracted similar image but still has to use the conventional technique of referring to an atlas in order to make a final determination. The determination of a subtype with use of an atlas relies on the experience and skills of the doctor and therefore has problems in accuracy and efficiency.

The present disclosure has been made in view of the above problem, and an example object thereof is to improve accuracy in automatic determination of a subtype carried out on the basis of a pathological image.

### Solution to Problem

A diagnosis assistance apparatus in accordance with an example aspect of the present disclosure includes at least one processor, the at least one processor carrying out: a first acquisition process of acquiring a first caption C from a first learned model, the first learned model being constructed by machine learning so as to generate, in a case where a pathological image is inputted, a caption C describing content of the pathological image in a predetermined format, the first caption C describing, in the predetermined format, content of a first pathological image to be subjected to diagnosis; a first evaluation process of evaluating a first similarity which is a similarity between (i) content of at least a part of a plurality of findings which are accumulated in a database and which represent, in writing in the predetermined format, a respective plurality of pathological subtypes and (ii) content of the first caption C; and an output process of outputting information pertaining to a subtype whose first similarity is evaluated to be the highest among those of the plurality of pathological subtypes.

A recording medium in accordance with an example aspect of the present disclosure is a non-transitory recording medium having recorded thereon a diagnosis assistance program for causing at least one processor to carry out:
a first acquisition process of acquiring a first caption from a first learned model, the first learned model being constructed by machine learning so as to generate, in a case where a pathological image is inputted, a caption describing content of the pathological image in a predetermined format, the first caption describing, in the predetermined format, content of a first pathological image to be subjected to diagnosis;
a first evaluation process of evaluating a first similarity which is a similarity between (i) content of at least a part of a plurality of findings which are accumulated in a database and which represent, in writing in the predetermined format, a respective plurality of pathological subtypes and (ii) content of the first caption; and
an output process of outputting information pertaining to a subtype whose first similarity is evaluated to be the highest among those of the plurality of pathological subtypes.

A diagnosis assistance method in accordance with an example aspect of the present disclosure includes: acquiring a first caption from a first learned model, the first learned model being constructed by machine learning so as to generate, in a case where a pathological image is inputted, a caption describing content of the pathological image in a predetermined format, the first caption describing, in the predetermined format, content of a first pathological image to be subjected to diagnosis; evaluating a first similarity which is a similarity between (i) content of at least a part of a plurality of findings which are accumulated in a database and which represent, in writing in the predetermined format, a respective plurality of pathological subtypes and (ii) content of the first caption; and outputting information pertaining to at least a subtype whose first similarity is evaluated to be the highest among those of the plurality of pathological subtypes.

### Advantageous Effects of Invention

According to an example aspect of the present disclosure, it is possible to improve accuracy in automatic determination of a subtype carried out on the basis of a pathological image.

### Brief Description of Drawings

Fig. 1 is a block diagram illustrating an example of a functional configuration of a diagnosis assistance apparatus in accordance with a first example embodiment of the present disclosure.
Fig. 2 is a view illustrating a function of a first learned model that generates a caption acquired by the diagnosis assistance apparatus.
Fig. 3 is table indicating information accumulated in a database used by the diagnosis assistance apparatus.
Fig. 4 is a flowchart illustrating an example of a flow of a diagnosis assistance method in accordance with the first example embodiment of the present disclosure.
Fig. 5 is a block diagram illustrating an example of a functional configuration of a diagnosis assistance apparatus in accordance with a second example embodiment of the present disclosure.
Fig. 6 is a view illustrating a function of a second learned model that generates feature information acquired by the diagnosis assistance apparatus.
Fig. 7 is table indicating information accumulated in a database used by the diagnosis assistance apparatus.
Fig. 8 is a table indicating an example of correspondence between (i) a subtype accumulated in the database and (ii) a first evaluation value and a second evaluation value calculated by the diagnosis assistance apparatus.
Fig. 9 is a flowchart illustrating an example of a flow of a diagnosis assistance method in accordance with the second example embodiment of the present disclosure.
Fig. 10 is a block diagram illustrating an example of a functional configuration of a diagnosis assistance apparatus in accordance with a third example embodiment of the present disclosure.
Fig. 11 is a flowchart illustrating an example of a flow of a diagnosis assistance method in accordance with the third example embodiment of the present disclosure.
Fig. 12 is a block diagram illustrating a hardware configuration of a computer functioning as each of the diagnosis assistance apparatuses in accordance with the example embodiments of the present disclosure.

### Example Embodiments

The following will exemplify embodiments of the present invention. However, the present invention is not limited to example embodiments described below, but may be altered in various ways by a skilled person within the scope of the claims. For example, the present invention can also encompass, in the scope of the present invention, any example embodiment derived by appropriately combining technical means employed in the example embodiments described below. Further, the present invention can also encompass, in the scope of the present invention, any example embodiment derived by appropriately omitting part of technical means employed in the example embodiments described below. Further, the effects mentioned in the example embodiments described below are examples of the effects expected in the example embodiments described below, and are not intended to define an extension of the present invention. That is, the present invention can also encompass, in the scope of the present invention, any example embodiments that do not bring about the effects mentioned in the example embodiments described below.

### [First example embodiment]

A first example embodiment which is an example of an embodiment of the present invention will be described in detail with reference to the drawings. The present example embodiment is a basic form of each example embodiment described later. Note that the scope of the application of each technical means employed in the present example embodiment is not limited to the present example embodiment. That is, each technical means employed in the present example embodiment can also be employed in other example embodiments included in the present disclosure to the extent that no particular technical obstruction occurs. In addition, each technical means illustrated in the drawings which are referred to for the description of the present example embodiment can also be employed in other example embodiments included in the present disclosure to the extent that no particular technical obstruction occurs.

### (Configuration of diagnosis assistance apparatus 1)

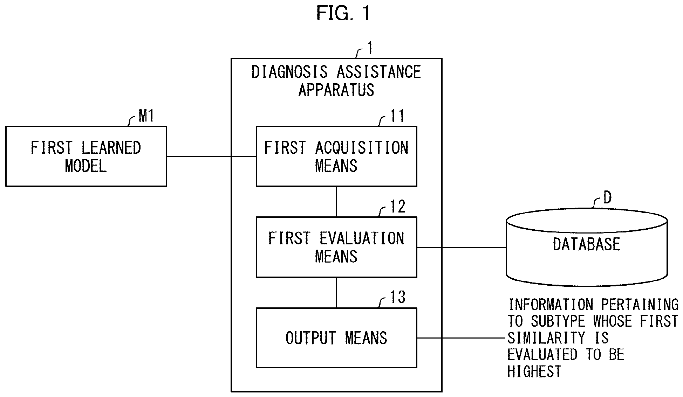

The following description will discuss a configuration of a diagnosis assistance apparatus 1 with reference to Fig. 1. Fig. 1 is a block diagram illustrating a configuration of the diagnosis assistance apparatus 1. The diagnosis assistance apparatus 1 includes a first acquisition means 11, a first evaluation means 12, and an output means 13 as illustrated in Fig. 1.

### · First acquisition means 11

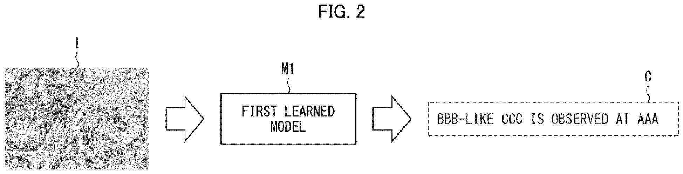

The first acquisition means 11 acquires, from a first learned model M1, a first caption C which describes, in a predetermined format, content of a first pathological image I to be subjected to diagnosis. The first learned model M1 is constructed by machine learning. Specifically, the first learned model M1 is constructed so as to generate, in a case where a pathological image I is inputted, a caption C which describes content of the pathological image I in the predetermined format as illustrated in Fig. 2. The first learned model M1 may be included in the diagnosis assistance apparatus 1 or may be provided in another apparatus that communicates with the diagnosis assistance apparatus 1.

### · First evaluation means 12

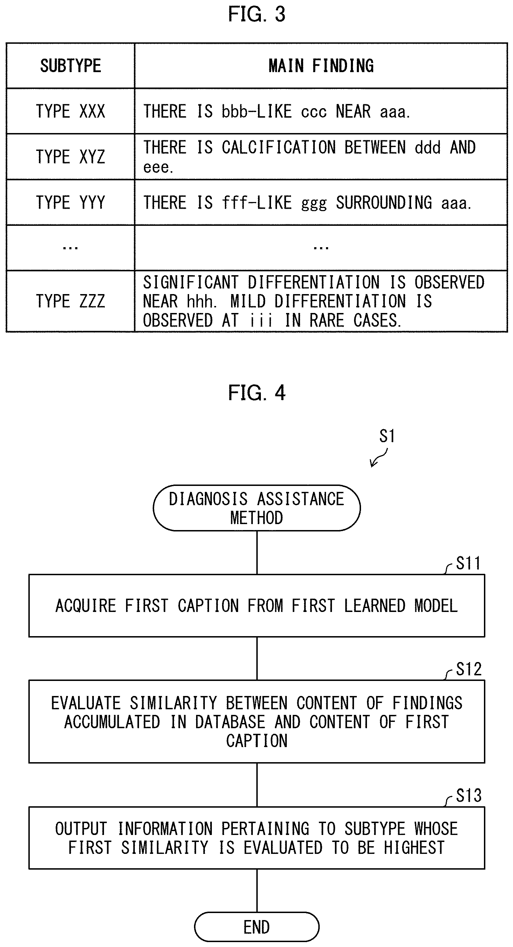

The first evaluation means 12 evaluates a first similarity, which is a similarity between content of at least a part of a plurality of findings accumulated in a database D and content of the first caption C. The "plurality of findings" means representation, in writing in the predetermined format (a format identical to that of the caption), of a respective plurality of pathological subtypes. The plurality of findings are each accumulated so as to be associated with a corresponding subtype as illustrated in Fig. 3. The "at least a part of a plurality of findings" encompasses a "finding related to the same portion among the plurality of findings", a "finding related to the same type of lesion among the plurality of findings", a "finding other than a clearly irrelevant finding among the plurality of findings", and the like. The first evaluation means 12 may be configured to represent a result of evaluation of the first similarity with use of a numerical value in a range of, for example, 0.0 to 1.0, or may be configured to represent the result of evaluation of the first similarity by a technique (e.g., use of a level such as major/medium/minor) other than use of a numerical value.

### · Output means 13

The output means 13 outputs information pertaining to a subtype whose first similarity is evaluated to be the highest among those of the plurality of pathological subtypes. The outputted information pertaining to the subtype is referred to by a doctor who performs diagnosis. The doctor who refers to the information pertaining to the subtype makes decisions in diagnosis while taking account of the information pertaining to the subtype.

### (Advantageous effect of diagnosis assistance apparatus 1)

The diagnosis assistance apparatus 1 described above employs a configuration in which the first evaluation means 12 evaluates the first similarity (similarity between the content of at least a part of the plurality of findings and the content of the first caption C). Further, the diagnosis assistance apparatus 1 employs a configuration in which the output means 13 outputs information pertaining to a subtype whose first similarity is evaluated to be the highest among those of the plurality of pathological subtypes. That is, the diagnosis assistance apparatus 1 compares the first caption (corresponding to a finding from the first pathological image) with the plurality of accumulated findings and presents to a user a finding that is the most similar to the first caption. Since the first learned model M1 has been constructed so as to output a caption in the predetermined format, content of a caption corresponding to an inputted pathological image depicts a feature of the pathological image more accurately. Further, since the findings for comparison are each written in the same predetermined format, similarity is evaluated more accurately. As such, the diagnosis assistance apparatus 1 in accordance with the present embodiment has an advantageous effect of making it possible to improve accuracy in automatic determination of a subtype carried out on the basis of a pathological image (and consequently to reduce the burden on the doctor in the task of referring to an atlas).

### (Flow of diagnosis assistance method S1)

The following description will discuss a flow of a diagnosis assistance method S1 with reference to Fig. 4. Fig. 4 is a flowchart illustrating a flow of the diagnosis assistance method S1. As illustrated in Fig. 4, the diagnosis assistance method S1 includes a first acquisition step S11, a first evaluation step S12, and an output step S13.

### · First acquisition step S11

The first acquisition step S11 is a step of acquiring, from the first learned model M1, a first caption C which describing, in a predetermined format, content of a first pathological image I to be subjected to diagnosis. For the acquisition of the first caption C, for example, the above diagnosis assistance apparatus 1 may be used.

### · First evaluation step S12

After the acquisition of the first caption C, the method proceeds to the first evaluation step S12. In the first evaluation step S12, a first similarity is evaluated. The first similarity is a similarity between content of at least a part of a plurality of findings accumulated in the database D and content of the first caption C. The evaluation of the first similarity may be carried out with use of the above diagnosis assistance apparatus 1 or with use of another apparatus.

### · Output step S13

After the evaluation of the first similarity, the method proceeds to the output step S13. The output step S13 is a step of outputting information pertaining to at least a subtype whose first similarity is evaluated to be the highest among those of the plurality of pathological subtypes. The output of the information pertaining to the subtype whose first similarity is evaluated to be the highest may be carried out with use of the above diagnosis assistance apparatus 1 or with use of another apparatus.

### (Advantageous effect of diagnosis assistance method S1)

As described above, the diagnosis assistance method S1 employs a configuration in which the first evaluation step S12 is a step of evaluating the first similarity (similarity between the content of at least a part of the plurality of findings and the content of the first caption C). Further, the diagnosis assistance method S1 employs a configuration in which the output step S13 is a step of outputting information pertaining to a subtype whose first similarity is evaluated to be the highest among those of the plurality of pathological subtypes. That is, in the diagnosis assistance method S1, the first caption (corresponding to a finding from the first pathological image) is compared with the plurality of accumulated findings, and a finding that is the most similar to the first caption is presented to a user. Since the first learned model M1 has been constructed so as to output a caption in the predetermined format, content of a caption corresponding to an inputted pathological image depicts a feature of the pathological image more accurately. Further, since the findings for comparison are each written in the same predetermined format, similarity is evaluated more accurately. As such, the diagnosis assistance method S1 in accordance with the present embodiment has an advantageous effect of making it possible to improve accuracy in automatic determination of a subtype carried out on the basis of a pathological image (and consequently to reduce the burden on the doctor in the task of referring to an atlas).

### [Second example embodiment]

A second example embodiment which is an example of an embodiment of the present invention will be described in detail with reference to the drawings. Note that constituent elements having the same functions as those described in the above-described example embodiment are denoted by the same reference numerals, and a description thereof will be omitted accordingly. Note that the scope of the application of each technical means employed in the present example embodiment is not limited to the present example embodiment. That is, each technical means employed in the present example embodiment can also be employed in other example embodiments included in the present disclosure to the extent that no particular technical obstruction occurs. In addition, each technical means illustrated in the drawings which are referred to for the description of the present example embodiment can also be employed in other example embodiments included in the present disclosure to the extent that no particular technical obstruction occurs.

### (Configuration of diagnosis assistance apparatus 1A)

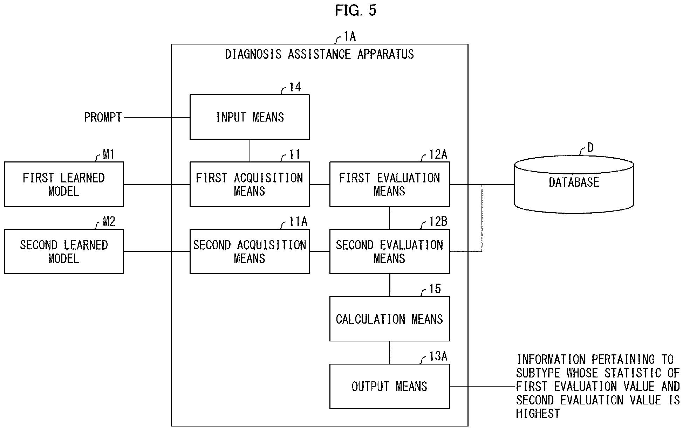

The following description will discuss a configuration of a diagnosis assistance apparatus 1A with reference to Fig. 5. Fig. 5 is a block diagram illustrating a configuration of the diagnosis assistance apparatus 1A. As illustrated in Fig. 5, the diagnosis assistance apparatus 1A in accordance with the present example embodiment includes a first acquisition means 11, which is similar to the one in the diagnosis assistance apparatus 1 in accordance with the above first example embodiment. The diagnosis assistance apparatus 1A in accordance with the present example embodiment further includes a first evaluation means 12A, a second acquisition means 11A, a second evaluation means 12B, an output means 13A, an input means 14, and a calculation means 15.

### · Input means 14

The input means 14 inputs a prompt to a first learned model M1 together with a first pathological image I, the prompt being related to output of a caption C. The input means 14 also inputs the first pathological image I to a second learned model M2. As a result of the input of the prompt to the first learned model M1, the first acquisition means 11 acquires, from the first learned model M1, a first caption C in a predetermined format that is based on the prompt. The "predetermined format" is a format which, as illustrated in Fig. 3, specifies a lesion (ccc, calcification, ggg, differentiation, etc.), a position of the legion (near aaa, between ddd and eee, surrounding aaa, near hhh, etc.), and a state and a degree of the legion (bbb-like, fff-like, significant, mild, etc.).

### · First evaluation means 12A

The first evaluation means 12A in accordance with the present example embodiment calculates a first evaluation value. The first evaluation value is a result of evaluation of a first similarity. That is, the first evaluation value is a numerical representation of the first similarity.

### · Second acquisition means 11A

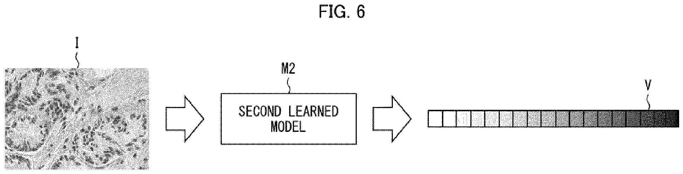

The second acquisition means 11A acquires first feature information from the second learned model M2. The first feature information is information indicating a feature of the first pathological image I. The second learned model M2 is constructed by machine learning. Specifically, the second learned model M2 is constructed so as to generate, in a case where a pathological image I is inputted, feature information indicating a feature of the pathological image I as illustrated in Fig. 6. The second learned model M2 used by the diagnosis assistance apparatus 1A in accordance with the present example embodiment is constructed by contrastive learning or an autoencoder. As such, the feature information outputted by the second learned model M2 is a feature vector V as illustrated in Fig. 6. Note that the second learned model M2 may be constructed by machine learning other than contrastive learning and an autoencoder. The feature information may be in a form other than the feature vector V.

### · Second evaluation means 12B

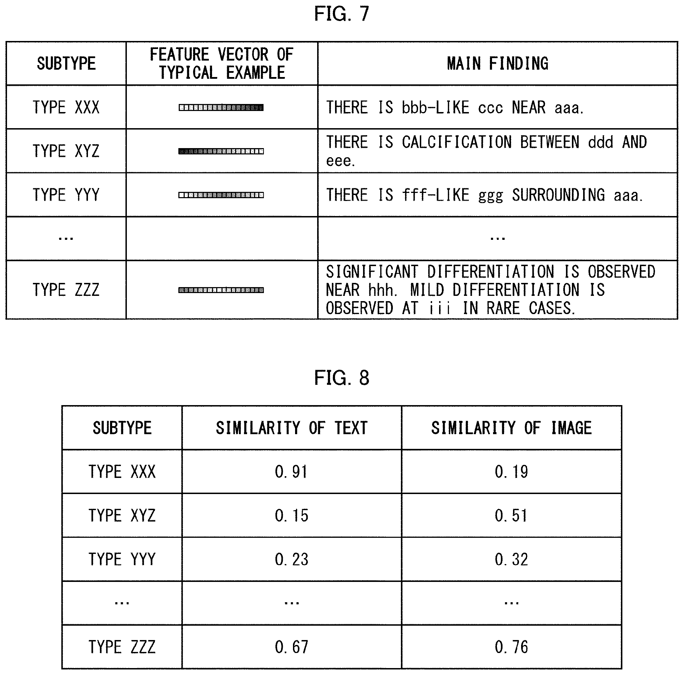

The second evaluation means 12B evaluates a second similarity. The second similarity is a similarity between (i) at least a part of a plurality of pieces of feature information which are accumulated in a database D and indicate respective features of a plurality of pathological images I respectively corresponding to a plurality of pathological subtypes and (ii) the first feature information. The plurality of pieces of feature information (feature vectors) are each accumulated so as to be associated with a corresponding subtype and a corresponding main finding as illustrated in Fig. 7. The database D in which the plurality of pieces of feature information are accumulated may be identical to or different from the database D described in the above first example embodiment. The second evaluation means 12B in accordance with the present example embodiment calculates a second evaluation value. The second evaluation value is a result of evaluation of the second similarity. The second evaluation means 12B in accordance with the present example embodiment calculates, as the second evaluation value, a cosine similarity between (i) at least a part of the plurality of feature vectors accumulated in the database D and (ii) a first feature vector, which is the first feature information. Note that the second evaluation means 12B may be configured to calculate, as the second evaluation value, a numerical value (e.g., a Euclidean distance, a Chebyshev distance, etc.) other than the cosine similarity.

The second evaluation value calculated by the second evaluation means 12B and the first evaluation value calculated by the first evaluation means 12A are each associated with any of the plurality of subtypes accumulated in the database D as illustrated in Fig. 8.

### · Calculation means 15

The calculation means 15 calculates a statistic of the first evaluation value and the second evaluation value. The "statistic" encompasses values obtained by addition, weighted addition, averaging, weighted averaging, selection, and the like. A value obtained by addition is a sum of the first evaluation value and the second evaluation value. A value obtained by averaging is an average of the first evaluation value and the second evaluation value. A value obtained by weighted averaging is an average of values respectively obtained by multiplying the first evaluation value and the second evaluation value by a weighting coefficient. A value obtained by selection is one of the first evaluation value and the second evaluation value which one is higher than the other.

### · Output means 13A

The output means 13A in accordance with the present example embodiment outputs information pertaining to a subtype having the highest statistic among the plurality of pathological subtypes. The "outputting" encompasses display of the information on a display section, output of audio of the information through a speaker, and transmission of a signal of the information to another apparatus through a communication module or the like. The output means 13A in accordance with the present example embodiment outputs two or more subtypes among the plurality of pathological subtypes in descending order of statistics. Note that the output means 13A may be configured to output at least one of the first evaluation value, the second evaluation value, and the statistic together with the subtypes.

### (Advantageous effect of diagnosis assistance apparatus 1A)

The diagnosis assistance apparatus 1A described above provides the same example advantage that is provided by the diagnosis assistance apparatus 1 in accordance with the above first example embodiment. That is, the diagnosis assistance apparatus 1A has an advantageous effect of making it possible to improve accuracy in automatic determination of a subtype carried out on the basis of a pathological image (and consequently to reduce the burden on the doctor in the task of referring to an atlas). Further, the diagnosis assistance apparatus 1A described above employs a configuration in which the second acquisition means 11A acquires the first feature information from the second learned model M2. Further, the diagnosis assistance apparatus 1A employs a configuration in which the second evaluation means 12B evaluates the second similarity (calculates the second evaluation value). Further, the diagnosis assistance apparatus 1A employs a configuration in which the output means 13A outputs information pertaining to a subtype having the highest statistic among the plurality of pathological subtypes. That is, the diagnosis assistance apparatus 1A outputs a subtype on the basis of two types of evaluation values. As such, the diagnosis assistance apparatus 1A has an advantageous effect of making it possible to further improve accuracy in automatic determination of a subtype carried out on the basis of a pathological image.

### (Flow of diagnosis assistance method S1A)

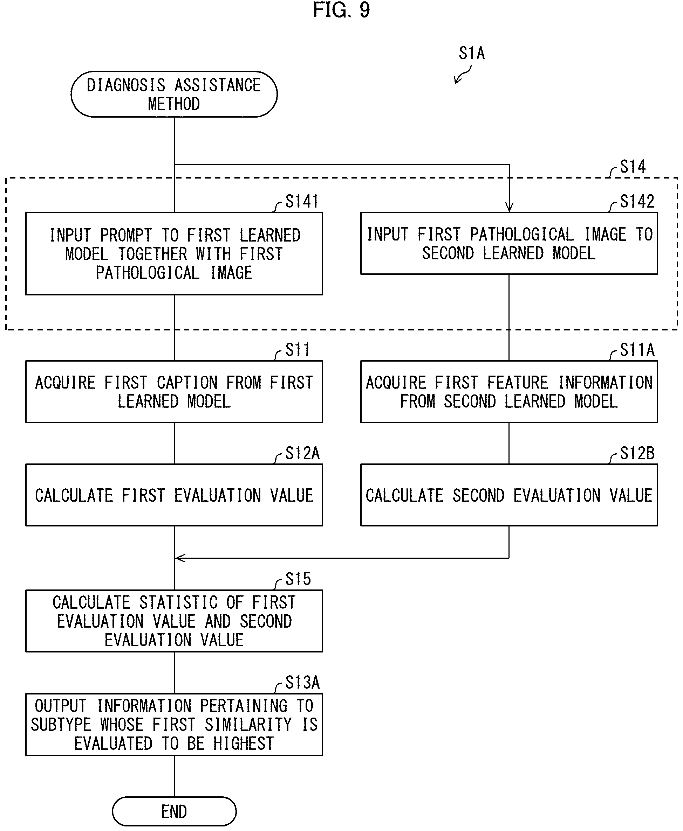

The following description will discuss a flow of a diagnosis assistance method S1A with reference to Fig. 9. Fig. 9 is a flowchart illustrating a flow of the diagnosis assistance method S1A. As illustrated in Fig. 9, the diagnosis assistance method S1A includes a first acquisition step S11, which is similar to the one in the diagnosis assistance method S1 in accordance with the above first example embodiment. The diagnosis assistance method S1A further includes a first evaluation step S12A, a second acquisition step S11A, a second evaluation step S12B, an output step S13A, an input step S14, and a calculation step S15.

### · Input step S14

The input step S14 includes a first input step S141 and a second input step S142. The first input step S141 is carried out before a first caption C is acquired. The first input step S141 is a step of inputting a prompt to the first learned model M1 together with a first pathological image I, the prompt being is related to output of a caption C. Before, after, or in parallel with the input of the prompt, the second input step S142 is carried out. The second input step S142 is a step of inputting the first pathological image I to the second learned model M2. For the input of the first pathological image I and the first caption C, for example, the above diagnosis assistance apparatus 1A may be used. As a result of the input of the prompt to the first learned model M1, the first caption C in a predetermined format that is based on the prompt is acquired from the first learned model M1 in the first acquisition step S11.

### · First evaluation step S12A

After the acquisition of the first caption C, the method proceeds to the first evaluation step S12A. In the first evaluation step S12A in accordance with the present example embodiment, a first evaluation value is calculated. The calculation of the first evaluation value may be carried out with use of the above diagnosis assistance apparatus 1A or with use of another apparatus.

### · Second acquisition step S11A

After, before, or in parallel with the calculation of the first evaluation value, the second acquisition step S11A is carried out. The second acquisition step S11A is a step of acquiring first feature information (a feature vector) from the second learned model M2. The acquisition of the first feature information may be carried out with use of the above diagnosis assistance apparatus 1A or with use of another apparatus.

### · Second evaluation step S12B

After the acquisition of the first feature information, the second evaluation step S12B is carried out. The second evaluation step S12B is a step of calculating, as a second evaluation value, a cosine similarity between (i) at least a part of a plurality of feature vectors accumulated in the database D and (ii) a first feature vector, which is the first feature information. The calculation of the second evaluation value may be carried out with use of the above diagnosis assistance apparatus 1A or with use of another apparatus.

### · Calculation step S15

After the calculation of the first evaluation value and the second evaluation value, the method proceeds to the calculation step S15. The calculation step S15 is a step of calculating a statistic of the first evaluation value and the second evaluation value. The calculation of the statistic may be carried out with use of the above diagnosis assistance apparatus 1A or with use of another apparatus.

### · Output step S13A

After the calculation of the statistic, the output step S13A is carried out. The output step S13A in accordance with the present example embodiment is a step of outputting information pertaining to a subtype having the highest statistic of the first evaluation value and the second evaluation value among the plurality of pathological subtypes. In the output step S 13A in accordance with the present example embodiment, two or more subtypes among the plurality of pathological subtypes are outputted in descending order of statistics. The output of the subtypes may be carried out with use of the above diagnosis assistance apparatus 1A or with use of another apparatus.

### (Advantageous effect of diagnosis assistance method S1A)

The diagnosis assistance method S1A described above provides the same example advantage that is provided by the diagnosis assistance method S1 in accordance with the above first example embodiment. That is, the diagnosis assistance method S1A has an advantageous effect of making it possible to improve accuracy in automatic determination of a subtype carried out on the basis of a pathological image (and consequently to reduce the burden on the doctor in the task of referring to an atlas). Further, the diagnosis assistance method S1A described above employs a configuration in which the first feature information is acquired from the second learned model M2 in the second acquisition step S11A. Further, the diagnosis assistance method S1A employs a configuration in which the second similarity is evaluated (the second evaluation value is calculated) in the second evaluation step S12B. Further, the diagnosis assistance method S1A employs a configuration in which information pertaining to a subtype having the highest statistic among the plurality of pathological subtypes is outputted in the output step S13A. That is, in the diagnosis assistance method S1A, a subtype is outputted on the basis of two types of evaluation values. As such, the diagnosis assistance method S1A has an advantageous effect of making it possible to further improve accuracy in automatic determination of a subtype carried out on the basis of a pathological image.

### [Third example embodiment]

A third example embodiment which is an example of an embodiment of the present invention will be described in detail with reference to the drawings. Note that constituent elements having the same functions as those described in the above-described example embodiments are denoted by the same reference numerals, and a description thereof will be omitted accordingly. Note that the scope of the application of each technical means employed in the present example embodiment is not limited to the present example embodiment. That is, each technical means employed in the present example embodiment can also be employed in other example embodiments included in the present disclosure to the extent that no particular technical obstruction occurs. In addition, each technical means illustrated in the drawings which are referred to for the description of the present example embodiment can also be employed in other example embodiments included in the present disclosure to the extent that no particular technical obstruction occurs.

### (Configuration of diagnosis assistance apparatus 1B)

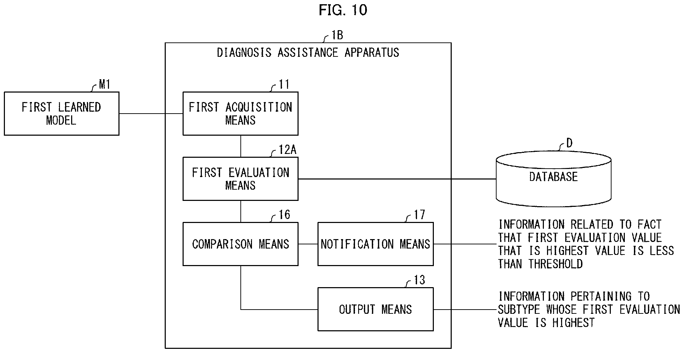

The following description will discuss a configuration of a diagnosis assistance apparatus 1B with reference to Fig. 10. Fig. 10 is a block diagram illustrating a configuration of the diagnosis assistance apparatus 1B. As illustrated in Fig. 10, the diagnosis assistance apparatus 1B in accordance with the present example embodiment includes a first acquisition means 11 and an output means 13, which are similar to those in the diagnosis assistance apparatus 1 in accordance with the above first example embodiment, and a first evaluation means 12A, which is similar to the one in the diagnosis assistance apparatus 1A in accordance with the above second example embodiment. The diagnosis assistance apparatus 1B further includes a comparison means 16 and a notification means 17. Note that the diagnosis assistance apparatus 1B may further include an input means 14, a second acquisition means 11A, a second evaluation means 12B, and a calculation means 15, which are similar to those in the diagnosis assistance apparatus 1A in accordance with the above second example embodiment.

### · Comparison means 16

The comparison means 16 compares one or more first evaluation values with a predetermined threshold. Note that in a case where the diagnosis assistance apparatus 1B further includes the second acquisition means 11A, the second evaluation means 12B, and the calculation means 15, the comparison means 16 may be configured to compare one or more statistics with a threshold.

### · Notification means 17

In a case where a first evaluation value that is the highest value among the one or more first evaluation values is less than the threshold, the notification means 17 provides notification of information related to that fact. The "information related to that fact (that a first evaluation value that is the highest value is less than a threshold)" encompasses (i) that the first evaluation value is less than the threshold (a state), (ii) that a normal evaluation cannot be made (an error), (iii) that at least one of a first pathological image I and a prompt is not appropriate (a cause), and (iv) that at least one of a first pathological image I and a prompt should be changed and a comparison should be retried (a solution). In a case where the comparison means 16 is configured to compare one or more statistics with a threshold, the notification means 17 may be configured such that, in a case where a statistic that is the highest value is less than the threshold, the notification means 17 provides notification of information related to that fact.

### (Advantageous effect of diagnosis assistance apparatus 1B)

The diagnosis assistance apparatus 1B described above provides the same example advantage that is provided by the diagnosis assistance apparatus 1 in accordance with the above first example embodiment. That is, the diagnosis assistance apparatus 1B has an advantageous effect of making it possible to improve accuracy in automatic determination of a subtype carried out on the basis of a pathological image (and consequently to reduce the burden on the doctor in the task of referring to an atlas). Further, the diagnosis assistance apparatus 1B described above employs a configuration in which the comparison means 16 compares one or more first evaluation values with a predetermined threshold. Further, the diagnosis assistance apparatus 1B employs a configuration in which, in a case where a first evaluation value that is the highest value is less than the threshold, the notification means 17 provides notification of information related to that fact. As such, the diagnosis assistance apparatus 1B has an advantageous effect of making it possible that, in a case where at least one of a first pathological image and a prompt which are inputted to the first learned model M1 has a problem, a user is made aware of that fact.

### (Flow of diagnosis assistance method S1B)

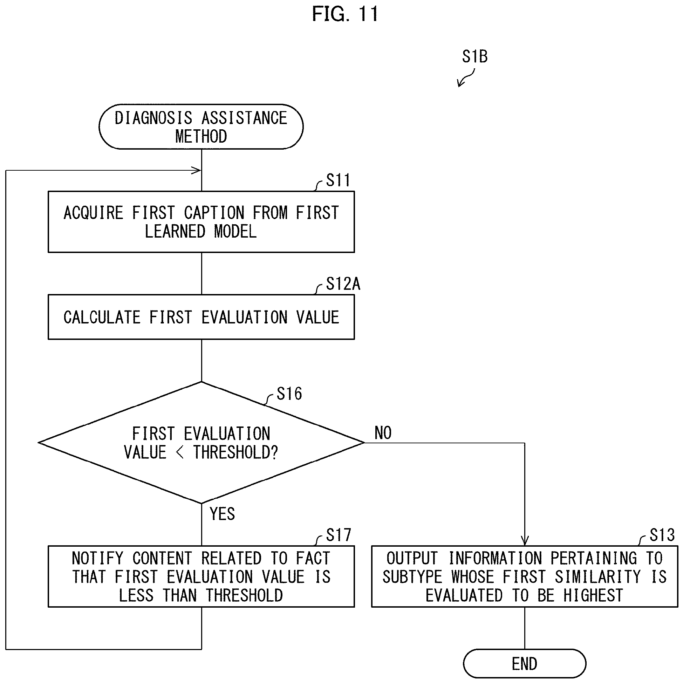

The following description will discuss a flow of a diagnosis assistance method S1B with reference to Fig. 11. Fig. 11 is a flowchart illustrating a flow of the diagnosis assistance method S1B. As illustrated in Fig. 11, the diagnosis assistance method S1B in accordance with the present example embodiment includes a first acquisition step S11 and an output step S13, which are similar to those in the diagnosis assistance method S1 in accordance with the above first example embodiment, and a first evaluation step S12A, which is similar to the one in the diagnosis assistance method S1A in accordance with the above second example embodiment. The diagnosis assistance method S1B in accordance with the present example embodiment further includes a comparison step S16 and a notification step S17 in addition to a first acquisition step S11 and an output step S13, which are similar to those in the diagnosis assistance method S1 in accordance with the above first example embodiment, and a first evaluation step S12A, which is similar to the one in the diagnosis assistance method S1A in accordance with the above second example embodiment. Note that the diagnosis assistance method S1B may further include an input step S14, a second acquisition step S11A, a second evaluation step S12B, and a calculation step S15, which are similar to those in the diagnosis assistance method S1A in accordance with the above second example embodiment.

### · Comparison step S16

After a first evaluation value is calculated, the comparison step S16 is carried out. The comparison step S16 is a step of comparing one or more first evaluation values with a predetermined threshold. The comparison of the one or more first evaluation values with the threshold may be carried out with use of the above diagnosis assistance apparatus 1A or with use of another apparatus. In a case where at least a first evaluation value that is the highest value among the one or more first evaluation values is not less than the threshold (S16: NO), the method proceeds to the output step S13. Note that in a case where the diagnosis assistance method S1B further includes the second acquisition step S11A, the second evaluation step S12B, and the calculation step S15, one or more statistics may be compared with a threshold in the comparison step S16.

### · Notification step S17

In a case where the first evaluation value that is the highest value among the one or more first evaluation values is less than the threshold (S16: YES), the notification step S17 is carried out. In the notification step S17, notification of information is provided which information is related to the fact that the first evaluation value that is the highest value is less than the threshold. The notification may be carried out with use of the above diagnosis assistance apparatus 1A or with use of another apparatus. After the notification of the information is provided, the method returns to the input step S14. In the input steps S14 carried out for the second and subsequent times, a change is made such that a first pathological image I and/or a prompt inputted to the first learned model M1 is/are different from the previously inputted one(s).

### (Advantageous effect of diagnosis assistance method S1B)

The diagnosis assistance method S1B described above provides the same example advantage that is provided by the diagnosis assistance method S1 in accordance with the above first example embodiment. That is, the diagnosis assistance method S1B has an advantageous effect of making it possible to improve accuracy in automatic determination of a subtype carried out on the basis of a pathological image (and consequently to reduce the burden on the doctor in the task of referring to an atlas). Further, the diagnosis assistance method S1B described above employs a configuration in which one or more first evaluation values are compared with a predetermined threshold in the comparison step S16. Further, the diagnosis assistance method S1B employs a configuration in which, in a case where a first evaluation value that is the highest value is less than the threshold, notification of information related to that fact is provided in the notification step S17. As such, the diagnosis assistance method S1B has an advantageous effect of making it possible that, in a case where at least one of a first pathological image and a prompt which are inputted to the first learned model M1 has a problem, a user is made aware of that fact.

### [Software Implementation Example]

Some or all of functions of each of the diagnosis assistance apparatuses 1, 1A, and 1B (hereinafter also referred to as "each apparatus above") can be realized by hardware such as an integrated circuit (IC chip) or can be alternatively realized by software.

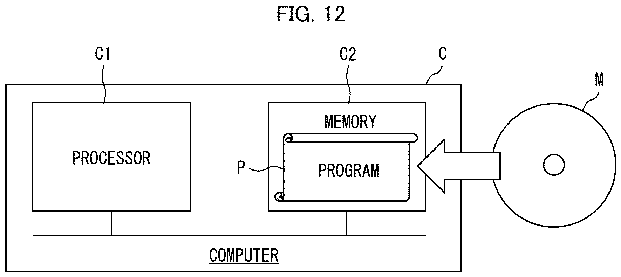

In the latter case, each apparatus above is realized by, for example, a computer that executes instructions of a program that is software realizing the foregoing functions. Fig. 12 illustrates an example of such a computer (hereinafter referred to as a "computer C"). Fig. 12 is a block diagram illustrating a hardware configuration of the computer C functioning as each apparatus above.

The computer C includes at least one processor C1 and at least one memory C2. The memory C2 has recorded therein a diagnosis assistance program P for causing the computer C to operate as each means above. The processor C1 of the computer C retrieves the diagnosis assistance program P from the at least one memory C2 and executes the diagnosis assistance program P, so that the functions of each apparatus above are realized.

The processor C1 may be, for example, a central processing unit (CPU), a graphic processing unit (CPU), a digital signal processor (DSP), a micro processing unit (MPU), a floating point number processing unit (FPU), a physics processing unit (PPU), a tensor processing unit (TPU), a quantum processor, a microcontroller, or a combination thereof. The memory C2 may be, for example, a flash memory, a hard disk drive (HDD), a solid state drive (SSD), or a combination thereof.

Note that the computer C may further include a random access memory (RAM) in which the diagnosis assistance program P is loaded at the time of execution and in which various data are temporarily stored. The computer C may further include a communication interface for carrying out transmission and reception of data to and from another apparatus. The computer C may further include an input/output interface for connecting the computer C to an input/output apparatus(es) such as a keyboard, a mouse, a display, and/or a printer.

The diagnosis assistance program P can also be recorded in a non-transitory tangible recording medium M from which the computer C can read the diagnosis assistance program P. Such a recording medium M can be, for example, a tape, a disk, a card, a semiconductor memory, a programmable logic circuit, or the like. The computer C can acquire the diagnosis assistance program P via the recording medium M. The diagnosis assistance program P can be transmitted via a transmission medium. The transmission medium may be, for example, a communication network, a broadcast wave, or the like. The computer C can acquire the diagnosis assistance program P also via the transmission medium.

### [Additional remark 1]

The present disclosure includes the techniques described in the supplementary notes below. Note, however, that the present invention is not limited to the techniques described in the supplementary notes below, but may be altered in various ways by a skilled person within the scope of the claims.

### (Supplementary note 1)

A diagnosis assistance apparatus, including:
a first acquisition means that acquires a first caption from a first learned model, the first learned model being constructed by machine learning so as to generate, in a case where a pathological image is inputted, a caption describing content of the pathological image in a predetermined format, the first caption describing, in the predetermined format, content of a first pathological image to be subjected to diagnosis;
a first evaluation means that evaluates a first similarity which is a similarity between (i) content of at least a part of a plurality of findings which are accumulated in a database and which represent, in writing in the predetermined format, a respective plurality of pathological subtypes and (ii) content of the first caption; and
an output means that outputs information pertaining to a subtype whose first similarity is evaluated to be the highest among those of the plurality of pathological subtypes.

### (Supplementary note 2)

The diagnosis assistance apparatus described in supplementary note 1, further including:
a second acquisition means that acquires first feature information from a second learned model, the second learned model being constructed by machine learning so as to generate, in a case where the pathological image is inputted, feature information indicating the pathological image, the first feature information indicating a feature of the first pathological image; and
a second evaluation means that evaluates a second similarity which is a similarity between (i) at least a part of a plurality of pieces of feature information which are accumulated in a database and indicate respective features of a plurality of pathological images respectively corresponding to the plurality of pathological subtypes and (ii) the first feature information,
wherein the output means outputs information pertaining to a subtype whose statistic of a first evaluation value and a second evaluation value is the highest among those of the plurality of pathological subtypes, the first evaluation value being a result of evaluation of the first similarity, the second evaluation value being a result of evaluation of the second similarity.

### (Supplementary note 3)

The diagnosis assistance apparatus described in supplementary note 1 or 2, wherein the output means outputs two or more subtypes among the plurality of pathological subtypes in descending order of statistics.

### (Supplementary note 4)

The diagnosis assistance apparatus described in any one of supplementary notes 1 to 3, further including an input means that inputs a prompt to the first learned model together with the first pathological image, the prompt being related to output of the caption,
wherein the first acquisition means acquires, from the first learned model, the first caption that is based on the prompt.

### (Supplementary note 5)

The diagnosis assistance apparatus described in supplementary note 2, further including:
a comparison means that compares one or more first evaluation values with a predetermined threshold, each of the one or more first evaluation values being the first evaluation value; and
a notification means that, in a case where a first evaluation value that is the highest value among the one or more first evaluation values is less than the threshold, provides notification of information related to that fact.

### (Supplementary note 6)

The diagnosis assistance apparatus described in supplementary note 2, wherein:
the feature information is a feature vector; and
the second evaluation means calculates, as the second evaluation value, a cosine similarity between (i) at least a part of a plurality of feature vectors accumulated in the database and (ii) a first feature vector which is the first feature information.

### (Supplementary note 7)

A diagnosis assistance program for causing a computer to function as the diagnosis assistance apparatus described in any one of supplementary notes 1 to 6,
the diagnosis assistance program causing the computer to function as the first acquisition means, the first evaluation means, and the output means.

### (Supplementary note 8)

A diagnosis assistance method, including:
a first acquisition step of acquiring a first caption from a first learned model, the first learned model being constructed by machine learning so as to generate, in a case where a pathological image is inputted, a caption describing content of the pathological image in a predetermined format, the first caption describing, in the predetermined format, content of a first pathological image to be subjected to diagnosis;
a first evaluation step of evaluating a first similarity which is a similarity between (i) content of at least a part of a plurality of findings which are accumulated in a database and which represent, in writing in the predetermined format, a respective plurality of pathological subtypes and (ii) content of the first caption; and
an output step of outputting information pertaining to at least a subtype whose first similarity is evaluated to be the highest among those of the plurality of pathological subtypes.

### (Supplementary note 9)

The diagnosis assistance method described in supplementary note 8, further including:
a second acquisition step of acquiring first feature information from a second learned model, the second learned model being constructed by machine learning so as to generate, in a case where the pathological image is inputted, feature information indicating the pathological image, the first feature information indicating a feature of the first pathological image; and
a second evaluation step of evaluating a second similarity which is a similarity between (i) at least a part of a plurality of pieces of feature information which are accumulated in a database and indicate respective features of a plurality of pathological images respectively corresponding to the plurality of pathological subtypes and (ii) the first feature information,
wherein in the output step, information pertaining to a subtype whose statistic of a first evaluation value and a second evaluation value is the highest among those of the plurality of pathological subtypes is outputted, the first evaluation value being a result of evaluation of the first similarity, the second evaluation value being a result of evaluation of the second similarity.

### (Supplementary note 10)

The diagnosis assistance method described in supplementary note 8 or 9, wherein in the output step, two or more subtypes among the plurality of pathological subtypes are outputted in descending order of statistics.

### (Supplementary note 11)

The diagnosis assistance method described in any one of supplementary notes 8 to 10, further including an input step of inputting a prompt to the first learned model together with the first pathological image, the prompt being related to output of the caption,
wherein in the first acquisition step, the first caption that is based on the prompt is acquired from the first learned model.

### (Supplementary note 12)

The diagnosis assistance method described in supplementary note 9, further including:
a comparison step of comparing one or more first evaluation values with a predetermined threshold, each of the one or more first evaluation values being the first evaluation value; and
a notification step of, in a case where a first evaluation value that is the highest value among the one or more first evaluation values is less than the threshold, providing notification of information related to that fact.

### (Supplementary Note 13)

The diagnosis assistance method described in supplementary note 9, wherein:
the feature information is a feature vector; and
in the second evaluation step, a cosine similarity between (i) at least a part of a plurality of feature vectors accumulated in the database and (ii) a first feature vector which is the first feature information is calculated as the second evaluation value.

### (Supplementary Note 14)

A diagnosis assistance program for causing a computer to function as the diagnosis assistance apparatus described in any one of supplementary notes 1 to 6,
the diagnosis assistance program causing the computer to function as the first acquisition means, the first evaluation means, and the output means.

### [Additional remark 2]

The techniques described in the supplementary notes below are within the scope of the present disclosure. Note, however, that the present invention is not limited to the techniques described in the supplementary notes below, but may be altered in various ways by a skilled person within the scope of the claims.

### (Supplementary note 1)

A diagnosis assistance apparatus, comprising at least one processor, the at least one processor carrying out:
a first acquisition process of acquiring a first caption from a first learned model, the first learned model being constructed by machine learning so as to generate, in a case where a pathological image is inputted, a caption describing content of the pathological image in a predetermined format, the first caption describing, in the predetermined format, content of a first pathological image to be subjected to diagnosis;
a first evaluation process of evaluating a first similarity which is a similarity between (i) content of at least a part of a plurality of findings which are accumulated in a database and which represent, in writing in the predetermined format, a respective plurality of pathological subtypes and (ii) content of the first caption; and
an output process of outputting information pertaining to a subtype whose first similarity is evaluated to be the highest among those of the plurality of pathological subtypes.

### (Supplementary note 2)

The diagnosis assistance apparatus described in supplementary note 1, wherein:
the at least one processor further carries out
   a second acquisition process of acquiring first feature information from a second learned model, the second learned model being constructed by machine learning so as to generate, in a case where the pathological image is inputted, feature information indicating the pathological image, the first feature information indicating a feature of the first pathological image, and
   a second evaluation process of evaluating a second similarity which is a similarity between (i) at least a part of a plurality of pieces of feature information which are accumulated in a database and indicate respective features of a plurality of pathological images respectively corresponding to the plurality of pathological subtypes and (ii) the first feature information; and
in the output process, the at least one processor outputs information pertaining to a subtype whose statistic of a first evaluation value and a second evaluation value is the highest among those of the plurality of pathological subtypes, the first evaluation value being a result of evaluation of the first similarity, the second evaluation value being a result of evaluation of the second similarity.

### (Supplementary note 3)

The diagnosis assistance apparatus described in supplementary note 1 or 2, wherein in the output process, the at least one processor outputs two or more subtypes among the plurality of pathological subtypes in descending order of statistics.

### (Supplementary note 4)

The diagnosis assistance apparatus described in any one of supplementary notes 1 to 3, wherein:
the at least one processor further carries out an input process of inputting a prompt to the first learned model together with the first pathological image, the prompt being related to output of the caption; and
in the first acquisition process, the at least one processor acquires, from the first learned model, the first caption that is based on the prompt.

### (Supplementary note 5)

The diagnosis assistance apparatus described in supplementary note 2, wherein the at least one processor further carries out:
a comparison process of comparing one or more first evaluation values with a predetermined threshold, each of the one or more first evaluation values being the first evaluation value; and
a notification process of, in a case where a first evaluation value that is the highest value among the one or more first evaluation values is less than the threshold, providing notification of information related to that fact.

### (Supplementary note 6)

The diagnosis assistance apparatus described in supplementary note 2, wherein:
the feature information is a feature vector; and
in the second evaluation step, the at least one processor calculates, as the second evaluation value, a cosine similarity between (i) at least a part of a plurality of feature vectors accumulated in the database and (ii) a first feature vector which is the first feature information.

### Reference Signs List

1, 1A, 1B: Diagnosis assistance apparatus
11: First acquisition means
11A: Second acquisition means
12, 12A: First evaluation means
12B: Second evaluation means
13, 13A: Output means
14: Input means
15: Calculation means
16: Comparison means
17: Notification means
C1: Processor
C2: Memory
C: Computer
C1: Processor
C2: Memory
D: Database
M: Recording medium
M1: First learned model
M2: Second learned model
P: Diagnosis assistance program
S1, S1A, S1B: Diagnosis assistance method
S11: First acquisition step
S11A: Second acquisition step
S12, S12A: First evaluation step
S12B: Second evaluation step
S13, S13A: Output step
S14: Input step
S141: First input step
S142: Second input step
S15: Calculation step
S16: Comparison step
S17: Notification step

## Claims

1. A diagnosis assistance apparatus, comprising:
a first acquisition section that acquires a first caption from a first learned model, the first learned model being constructed by machine learning so as to generate, in a case where a pathological image is inputted, a caption describing content of the pathological image in a predetermined format, the first caption describing, in the predetermined format, content of a first pathological image to be subjected to diagnosis;
a first evaluation section that evaluates a first similarity which is a similarity between (i) content of at least a part of a plurality of findings which are accumulated in a database and which represent, in writing in the predetermined format, a respective plurality of pathological subtypes and (ii) content of the first caption; and
an output section that outputs information pertaining to a subtype whose first similarity is evaluated to be the highest among those of the plurality of pathological subtypes.

2. The diagnosis assistance apparatus according to claim 1, further comprising:
a second acquisition section that acquires first feature information from a second learned model, the second learned model being constructed by machine learning so as to generate, in a case where the pathological image is inputted, feature information indicating the pathological image, the first feature information indicating a feature of the first pathological image; and
a second evaluation section that evaluates a second similarity which is a similarity between (i) at least a part of a plurality of pieces of feature information which are accumulated in a database and indicate respective features of a plurality of pathological images respectively corresponding to the plurality of pathological subtypes and (ii) the first feature information,
wherein the output section outputs information pertaining to a subtype whose statistic of a first evaluation value and a second evaluation value is the highest among those of the plurality of pathological subtypes, the first evaluation value being a result of evaluation of the first similarity, the second evaluation value being a result of evaluation of the second similarity.

3. The diagnosis assistance apparatus according to claim 2, wherein the output section outputs two or more subtypes among the plurality of pathological subtypes in descending order of statistics.

4. The diagnosis assistance apparatus according to claim 1, further comprising an input section that inputs a prompt to the first learned model together with the first pathological image, the prompt being related to output of the caption,
wherein the first acquisition section acquires, from the first learned model, the first caption that is based on the prompt.

5. The diagnosis assistance apparatus according to claim 2, further comprising:
a comparison section that compares one or more first evaluation values with a predetermined threshold, each of the one or more first evaluation values being the first evaluation value; and
a notification section that, in a case where a first evaluation value that is the highest value among the one or more first evaluation values is less than the threshold, provides notification of information related to that fact.

6. The diagnosis assistance apparatus according to claim 2, wherein:
the feature information is a feature vector; and
the second evaluation section calculates, as the second evaluation value, a cosine similarity between (i) at least a part of a plurality of feature vectors accumulated in the database and (ii) a first feature vector which is the first feature information.

7. A diagnosis assistance program for causing a computer to function as a diagnosis assistance apparatus recited in claim 1,
the diagnosis assistance program causing the computer to function as the first acquisition section, the first evaluation section, and the output section.

8. A diagnosis assistance method, comprising:
a first acquisition step of acquiring a first caption from a first learned model, the first learned model being constructed by machine learning so as to generate, in a case where a pathological image is inputted, a caption describing content of the pathological image in a predetermined format, the first caption describing, in the predetermined format, content of a first pathological image to be subjected to diagnosis;
a first evaluation step of evaluating a first similarity which is a similarity between (i) content of at least a part of a plurality of findings which are accumulated in a database and which represent, in writing in the predetermined format, a respective plurality of pathological subtypes and (ii) content of the first caption; and
an output step of outputting information pertaining to at least a subtype whose first similarity is evaluated to be the highest among those of the plurality of pathological subtypes.
